(19) Europäisches Patentamt
European Patent Office
Office européen des brevets

(11) Veröffentlichungsnummer: **0 180 548**
**B1**

(12) # EUROPÄISCHE PATENTSCHRIFT

(45) Veröffentlichungstag der Patentschrift: **28.06.89**

(51) Int. Cl.$^4$: **C 09 D 7/12**, C 08 K 5/34, C 07 D249/20

(21) Anmeldenummer: **85810487.0**

(22) Anmeldetag: **28.10.85**

(54) **Gegen Lichteinwirkung stabilisierte Ueberzugsmittel.**

(30) Priorität: **01.11.84 CH 5220/84**
**01.02.85 CH 453/85**

(43) Veröffentlichungstag der Anmeldung: **07.05.86 Patentblatt 86/19**

(45) Bekanntmachung des Hinweises auf die Patenterteilung: **28.06.89 Patentblatt 89/26**

(84) Benannte Vertragsstaaten: **BE DE FR GB IT NL SE**

(56) Entgegenhaltungen:
**FR-A- 2 383 464**
**US-A- 3 766 205**
**US-A- 3 936 418**
**US-A- 4 344 830**
**CHEMICAL ABSTRACTS, Band 103, Nr. 16, 21. Oktober 1985, Seite 41, Zusammenfassung Nr. 124474x, Columbus, Ohio, US; & JP-A-60 71 663 (TORAY INDUSTRIES INC.) 23-04-1985**
**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Slongo, Mario, Dr.**
**Sägetrainweg 553**
**CH-1712 Tafers (CH)**
Erfinder: **Rembold, Manfred, Dr.**
**Im Aeschfeld 21**
**CH-4147 Aesch (CH)**

**EP 0 180 548 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Ueberzugsmittel, die Kryptolichtschutzmittel enthalten. Solche Verbindungen werden durch die Einwirkung von kurzwelligem Licht aktiviert, indem sie photochemisch in Lichtschutzmittel umgewandelt werden. Es handelt sich dabei um bestimmte Derivate von 2-(2-Hydroxyphenyl)-benztriazolen mit blockierter Hydroxylgruppe.

Es wurde von Olson und Schroeter (J. Appl. Pol. Science 22 (1978), 2165-72) bereits vorgeschlagen, die phenolische Hydroxylgruppe von 2-(2-Hydroxyphenyl)-benztriazolen mit chemischen Schutzgruppen zu blockieren, die später wieder abspaltbar sind, beispielsweise durch Hydrolyse, Photolyse oder durch Erwärmen. Diese blockierten Benztriazolderivate absorbieren nicht im UV-Bereich von 330-400 nm und stören dadurch nicht die Photopolymerisation von UV-härtbaren Massen. Wegen ihrer veränderten UV-Absorption (ihr Absorptionsmaximum liegt bei etwa 290-310 nm) sind sie aber als Lichtschutzmittel nicht brauchbar und müssen daher nach der Photopolymerisation in die als Lichtschutzmittel bekannten Benztriazole mit freier OH-Gruppe umgewandelt werden. Die genannten Autoren untersuchten die Möglichkeit zur Blockierung der OH-Gruppe durch Silylierung, durch Carbamoylierung und in einer späteren Arbeit (J. Appl. Pol. Science 28 (1983), 1159-65) auch durch Veretherung, Veresterung und Sulfonylierung. Hierbei treten verschiedene Probleme auf wie ungenügende Rückspaltung oder Verfärbung durch Bildung von Nebenprodukten. Als beste Methode wird die O-Sulfonylierung und photochemische Rückspaltung beschrieben. Bei dieser Rückspaltung erfolgt im wesentlichen eine photochemische Fries-Umlagerung zu den ortho-Sulfonylphenolen. Dies ist möglich, weil alle untersuchten Verbindungen in ortho-Position (zur OH-Gruppe) unsubstituiert sind.

Es wurde nun gefunden, dass man bei Verwendung von 2-(2-Hydroxyphenyl)-benztriazolen, die sowohl in para- wie in ortho-Position zur OH-Gruppe substituiert sind, durch Blockierung der OH-Gruppe zu Kryptolichtschutzmitteln gelangt, deren Absorptionsmaximum ebenfalls bei 290-310 nm liegt und die trotz der Substitution in para- und ortho-Position photochemisch in Verbindungen mit freier OH-Gruppe rückverwandelt werden können. Sie besitzen gegenüber den Verbindungen mit unsubstituierter ortho-Position eine höhere Beständigkeit im Dunkeln sowie eine höhere Hydrolysenbeständigkeit. Dies ist wichtig für die technische Anwendung, da Lacke oft lange Zeit gelagert werden.

In der US-A 3,936,418 wurden auch schon 2-(2-Hydroxyphenyl)-benztriazole beschrieben, deren OH-Gruppen durch Veresterung blockiert sind und die in para- und ortho-Position zur OH-Gruppe substituiert sind. In diesen Verbindungen geschieht die Veresterung durch eine Dicarbonsäure und die resultierenden Verbindungen sind in organischen Lösungsmitteln nur ungenügend löslich um in Lacken verwendet zu werden.

Die erfindungsgemässen Kryprolichtschutzmittel sind von Vorteil gegenüber den bekannten Benztriazol-Lichtschutzmitteln mit freier OH-Gruppe, wenn das Ueberzugsmittel vor oder während der Applikation in Kontakt mit Metallen steht. Alle 2-(2-Hydroxyphenyl)-benztriazole bilden mit Metallionen Komplexe, die oft farbig sind (z. B. im Falle von Cu ; Ni, Co) und dann eine Verfärbung des Ueberzugsmittels bewirken können. Ein anderes interessantes Einsatzgebiet für Kryptolichtschutzmittel sind oxydativ trocknende Ueberzugsmittel, die als Härtungskatalysator (Sikkativ) organische Metallverbindungen enthalten. In Gegenwart von freien 2-(2-Hydroxyphenyl)-benztriazolen werden diese Metallverbindungen koordinativ an das Benztriazol gebunden und stehen dann als Härtungskatalysatoren nicht mehr zur Verfügung.

Gegenstand der Erfindung sind Ueberzugsmittel, enthaltend als Kryptolichtschutzmittel eine Verbindung der Formel I oder II,

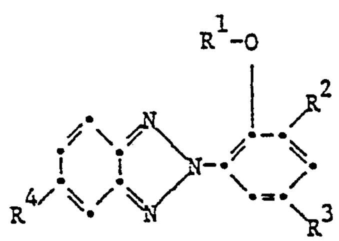

(I)

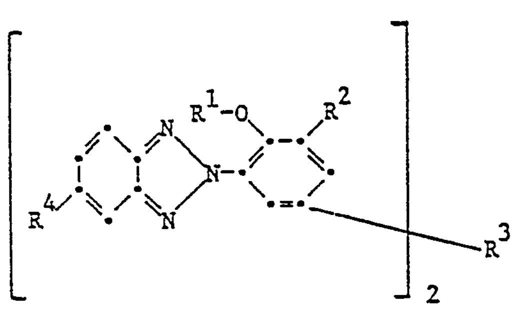

(II)

worin

$R^1$ eine Acylgruppe der Formel $—CO—R^5$, eine Sulfonylgruppe der Formel $—SO_2—R^6$ oder eine Phosphorylgruppe der Formel $—P(O)_r(R^{14})(R^{15})$ bedeutet,

$R^2$ $C_1$-$C_{12}$-Alkyl, $C_5$-$C_{12}$-Cycloalkyl, Phenyl, $C_7$-$C_9$-Phenylalkyl, $C_3$-$C_5$-Alkenyl oder Halogen bedeutet,

$R^3$ in Formel I $C_1$-$C_{12}$-Alkyl, $C_5$-$C_{12}$-Cycloalkyl, Phenyl, $C_7$-$C_9$-Phenylalkyl, Halogen oder eine Gruppe der Formel $—(CH_2)_n—COOR^9$ oder $—(CH_2)_n—CO—N(R^{10})(R^{11})$ bedeutet und in Formel II einen zweitwertigen Rest der Formeln $—(CH_2)_n—CO—O—(CH_2)_q—O—CO—(CH_2)_n—$, $—(CH_2)_n—CO—O—(CH_2CH_2O)_p—CO—(CH_2)_n—$, $—(CH_2)_n—CO—NH—R^{12}—NH—CO—(CH_2)_n—$ oder

$$-(CH_2)_n-CO-O-CH_2-\underset{\displaystyle OR^{13}}{\underset{|}{CH}}-CH_2-O-(CH_2)_q-O-CH_2-\underset{\displaystyle OR^{13}}{\underset{|}{CH}}-CH_2-O-CO-(CH_2)_n-$$

bedeutet,

$R^4$ Wasserstoff, Halogen, $C_1$-$C_8$-Alkyl, $C_7$-$C_9$-Phenylalkyl, $C_1$-$C_8$-Alkoxy oder $C_2$-$C_8$-Alkoxycarbonyl bedeutet,

$R^5$ $C_1$-$C_{18}$-Alkyl, $C_2$-$C_{18}$-Alkenyl, $—CH_2—CO—CH_3$, Phenyl, durch $C_1$-$C_{12}$-Alkyl, $C_1$-$C_4$-Alkoxy oder Benzoyl substituiertes Phenyl, $C_7$-$C_{12}$-Arylalkyl oder $C_1$-$C_{12}$-Alkoxy bedeutet,

$R^6$ $C_1$-$C_{12}$-Alkyl, $C_6$-$C_{10}$-Aryl oder $C_7$-$C_{18}$-Alkylaryl bedeutet,

$R^9$ Wasserstoff, $C_1$-$C_{12}$-Alkyl oder eine Gruppe der Formel $—(CH_2CH_2O)_p—R^1$ bedeutet,

$R^{10}$ und $R^{11}$ unabhängig voneinander Wasserstoff, $C_1$-$C_{12}$-Alkyl, das durch O oder N unterbrochen sein kann, $C_5$-$C_{12}$-Cycloalkyl, $C_7$-$C_9$-Phenylalkyl, $C_3$-$C_5$-Alkenyl, Phenyl oder einen 2,2,6,6-Tetramethyl-4-piperidinyl-Rest bedeuten, oder $R^{10}$ und $R^{11}$ zusammen $C_4$-$C_6$-Alkylen, -Oxaalkylen oder -Azaalkylen bedeuten,

$R^{12}$ $C_1$-$C_{12}$-Alkylen, das durch 1-3 O-Atome unterbrochen sein kann, bedeutet,

$R^{13}$ $C_1$-$C_{12}$-Alkyl oder $C_6$-$C_{10}$-Aryl bedeutet,

$R^{14}$ und $R^{15}$ unabhängig voneinander $C_1$-$C_{12}$-Alkoxy, Phenoxy, $C_1$-$C_{12}$-Alkyl, Cyclohexyl, Benzyl, Phenyl oder Tolyl bedeuten,

n 1 oder 2,

p eine Zahl von 1 bis 10,

q eine Zahl von 2 bis 12 und

r 0 oder 1 ist.

$R^1$ als einwertige Acylgruppe kann z. B. Acetyl, Propionyl, Butyryl, Valerianyl, Capronyl (n-Hexanoyl), 2-Ethylhexanoyl, Capryloyl (n-Octanoyl), Caprionyl (n-Decanoyl), Lauroyl (n-Dodecanoyl), Palmityl (n-Hexadecanoyl), Stearoyl (n-Octadecanoyl), Acryloyl, Methacryloyl, Crotonyl, Oleyl, Linoleyl, Benzoyl, 3-Methylbenzoyl, 4-tert.-Butylbenzoyl, 4-Hexylbenzoyl, 4-Dodecylbenzoyl, 3- oder 4-Methoxy- oder Ethoxybenzoyl, 4-Benzoyl-benzoyl, Phenylacetyl, Phenylpropionyl, Naphthylacetyl, Methoxycarbonyl, Ethoxycarbonyl, Butoxycarbonyl, Octyloxycarbonyl oder Dodecyloxycarbonyl sein Beispiele für $R^1$ als Sulfonylgruppe sind die Gruppen Methyl-, tert.-Butyl-, Octyl-, Dodecyl-, Phenyl-, Tolyl-, Naphthyl-, 4-Nonylphenyl-, 4-Dodecylphenyl- oder Mesitylensulfonyl.

$R^2$, $R^3$, $R^4$, $R^9$, $R^{10}$, $R^{11}$, $R^{13}$, $R^{14}$, $R^{15}$ als Alkyl können innerhalb der angegebenen C-Anzahl z. B. Methyl, Ethyl, Propyl, Isopropyl, n-Butyl, Isobutyl, sec.-Butyl, tert.-Butyl, Pentyl, Hexyl, Heptyl, Octyl, Decyl oder Dodecyl sein.

$R^2$ und $R^3$ sind bevorzugt verzweigte Alkylgruppen.

$R^{10}$ und $R^{11}$ als durch O oder N unterbrochenes Alkyl können z. B. 2-Butoxyethyl, 3-Methoxypropyl, 3-Ethoxypropyl, 3-Isopropoxypropyl-3-Butylaminopropyl oder 3-Ethylaminopropyl sein.

$R^2$, $R^{10}$ und $R^{11}$ als Alkenyl können z. B. Allyl, Methallyl oder 2-Butenyl sein.

$R^{14}$ und $R^{15}$ als Alkoxy können z. B. Methoxy, Ethoxy, Isopropoxy, Butoxy, Hexyloxy, Octyloxy, Decyloxyl oder Dodecyloxy sein.

$R^2$ und $R^3$ als Halogen können Fluor, Chlor, Brom sein, insbesondere Chlor.

$R^2$, $R^3$, $R^{10}$ und $R^{11}$ als Cycloalkyl können z. B. Cyclopentyl, Cyclohexyl, Methylcyclohexyl, Cyclooctyl oder Cyclododecyl sein.

$R^2$, $R^3$, $R^{10}$ und $R^{11}$ als Phenylalkyl können z. B. Benzyl, $\alpha$-Methylbenzyl, $\alpha$-Dimethylbenzyl oder 2-Phenylethyl sein. $R^2$ und $R^3$ als Phenylalkyl sind bevorzugt $\alpha$-Dimethylbenzyl.

$R^4$ als Alkoxy oder Alkoxycarbonyl kann z. B. Methoxy, Ethoxy, Isopropoxy, Butoxy, Hexyloxy, Octyloxy, Methoxycarbonyl, Ethoxycarbonyl, Butoxycarbonyl oder Pentyloxycarbonyl sein. Bevorzugt sind $C_1$-$C_4$-Alkoxy- oder Alkoxycarbonylreste.

Wenn $R^{10}$ und $R^{11}$ zusammen Alkylen, Oxa- oder Azaalkylen bedeuten, so bilden sie zusammen mit dem N-Atom, an das sie gebunden sind, einen heterocyclischen Ring, beispielsweise einen Pyrrolidin-, Piperidin-, Morpholin-, Piperazin- oder Hexamethyleniminring.

$R^{12}$ kann z. B. 1,2-Ethylen, Tri-, Tetra-, Penta-, Hexa-, Octa- oder Dodecamethylen, 2,4,4-Trimethylhexamethylen, 3-Oxaheptamethylen oder 3,6-Dioxa-decamethylen sein.

Bevorzugt sind Verbindungen der Formel I, worin

$R^1$ eine Gruppe der Formel $—CO—R^5$, $—SO_2—R^6$ oder $—P(O)(R^{14})(R^{15})$

$R^2$ $C_1$-$C_{12}$-Alkyl, Cyclohexyl oder $C_7$-$C_9$-Phenylalkyl,
$R^3$ $C_1$-$C_{12}$-Alkyl, Cyclohexyl, $C_7$-$C_9$-Phenylalkyl oder eine Gruppe $—CH_2CH_2\ COOR^9$
$R^4$ Wasserstoff, Methyl oder Chlor,
$R^5$ $C_1$-$C_{12}$-Alkyl, $C_2$-$C_4$-Alkenyl, Phenyl, Benzyl oder Naphthylmethyl,
$R^6$ Methyl, Phenyl oder $C_7$-$C_{18}$-Alkylphenyl,
$R^9$ $C_1$-$C_{12}$-Alkyl und
$R^{14}$ und $R^{15}$ unabhängig voneinander $C_1$-$C_4$-Alkoxy, Methyl oder Phenyl bedeuten.

Bevorzugt ist darin $R^1$ eine Gruppe $—CO—R^5$ oder $—SO_2—R^6$, $R^2$ $C_1$-$C_8$-Alkyl oder α-Dimethylbenzyl, $R^3$ $C_1$-$C_8$-Alkyl, α-Dimethylbenzyl oder eine Gruppe $—CH_2CH_2COOR^9$ und $R^4$ Wasserstoff oder Chlor.

Besonders bevorzugt sind solche Verbindungen der Formel I, worin $R^1$ eine Gruppe $—CO—R^5$ oder $—SO_2—R^6$ ist, $R^2$ $C_1$-$C_8$-Alkyl oder α-Dimethylbenzyl ist, $R^3$ $C_1$-$C_8$-Alkyl, α-Dimethylbenzyl oder $—CH_2CH_2COOR^9$ ist, $R^4$ Wasserstoff oder Chlor ist, $R^5$ $C_1$-$C_{12}$-Alkyl, $C_2$-$C_4$-Alkenyl, Phenyl, Benzyl oder Naphthylmethyl bedeutet, $R^6$ Methyl, Phenyl oder $C_7$-$C_{18}$-Alkylphenyl bedeutet und $R^9$ $C_1$-$C_{12}$-Alkyl ist.

Bevorzugt sind weiterhin Verbindungen der Formel II, worin $R^1$ eine Gruppe $—CO—R^5$ oder, $—SO_2—R^6$ ist, $R^2$ $C_1$-$C_8$-Alkyl oder α-Dimethylbenzyl bedeutet, $R^3$ eine Gruppe der Formel $—CH_2CH_2COO—(CH_2)_q—OCOCH_2CH_2—$ oder $—CH_2CH_2CONH—R^{12}—NHCOCH_2CH_2—$ bedeutet, $R^4$ Wasserstoff oder Chlor ist, $R^5$ $C_1$-$C_{12}$-Alkyl, $C_2$-$C_4$-Alkenyl, Phenyl, Benzyl oder Naphthylmethyl bedeutet, $R^6$ Methyl, Phenyl oder $C_7$-$C_{18}$-Alkylphenyl bedeutet, $R^{12}$ $C_2$-$C_8$-Alkylen und q eine Zahl von 2 bis 8 ist.

Diese Verbindungen sind zum Teil bekannte Verbindungen, zum Teil sind sie neue Verbindungen. Sie können prinzipiell aus den entsprechenden Hydroxylverbindungen ($R^1 = H$) durch Veresterung der Hydroxylgruppe nach den üblichen Methoden der Acylierung von phenolischen OH-Gruppen hergestellt werden, beispielsweise durch Reaktion mit Carbonsäureanhydriden, Carbonsäurechloriden, Sulfonsäurechloriden oder Chlorphosphaten. Beispiele hierfür werden in den anschliessenden Herstellungsbeispielen gegeben. Die als Ausgangsmaterial verwendeten Hydroxylverbindungen sind z. T. im Handel erhältlich oder können nach den zur Herstellung von 2-(2-Hydroxyphenyl)-benztriazolen allgemein bekannten Methoden hergestellt werden.

Neue Verbindungen sind z. B. diejenigen Verbindungen der Formel I, worin $R^3$ eine Gruppe $—(CH_2)_n—COOR^9$ oder $—(CH_2)_n—CON(R^{10})\ (R^{11})$ ist sowie Verbindungen der Formel II, worin $R^3$ eine zweiwertige Gruppe der Formeln $—(CH_2)_n—CO—O—(CH_2)_q—O—CO—(CH_2)_n—$, $—(CH_2)_n—CO—O—(CH_2CH_2O)_p—CO—(CH_2)_n—$, $—(CH_2)_n—CO—NH—R^{10}—NH—CO—(CH_2)—$ oder

$$-(CH_2)_n-CO-O-CH_2-\underset{\displaystyle OR^{11}}{\underset{|}{CH}}-CH_2-O-(CH_2)_q-O-CH_2-\underset{\displaystyle OR^{11}}{\underset{|}{CH}}-CH_2-O-CO-(CH_2)_n-$$

bedeutet.

Beispiele für einzelne Verbindungen der Formel I sind :

2-(2-Acetoxy-3-methyl-5-tert.-butyl-phenyl)-benztriazol
2-[2-Acetoxy-3,5-di(tert.-butyl)-phenyl]-benztriazol
2-(2-Propionyloxy-3-isopropyl-5-tert.-butyl-phenyl)-benztriazol
2-(2-Butyroyloxy-3-sec.-butyl-5-tert.-butyl-phenyl)-benztriazol
2-[2-Hexanoyloxy-3,5-di(tert.-pentyl)-phenyl]-benztriazol
5-Chlor-2-[2-octanoyloxy-3,5-di(tert.-butyl)-phenyl]-benztriazol
5-Methyl-2-(2-benzoyloxy-3,5-dimethyl-phenyl)-benztriazol
5-Methoxy-2-[2-(4-chlorbenzoyloxy)-3,5-di(tert.-butyl)-phenyl]-benztriazol
2-[2-p-Toluolsulfonyloxy-3,5-di(tert.-butyl)-phenyl]-benztriazol
2-[2-Dodecylsulfonyloxy-3,5-di(1,1-dimethylbenzyl)-phenyl]-benztriazol
2-[2-(p-Dodecylbenzolsulfonyloxy)-3-sec.-butyl-5-tert.-butylphenyl]-benztriazol
β-[3-(Benztriazol-2-yl)-4-acetoxy-5-tert.-butyl-phenyl]-propionsäuremethylester
[3-(Benztriazol-2-yl)-4-p-toluolsulfonyloxy-5-tert.-butyl-phenyl]-essigsäure-butylester
β-[3-(Benztriazol-2-yl)-4-benzoyloxy-5-cyclohexylphenyl]-propionsäure-2-butoxyethylester.

Beispiele für einzelne Verbindungen der Formel II sind :

Di-[3-(Benztriazol-2-yl)-4-acetoxy-5-tert.-butyl-phenyl-essigsäure-ester] von Diethylenglykol
Di-[β -(3-Benztriazol-2-yl)-4-p-toluolsulfonyloxy-5-sec.-butylphenyl)-propionsäureester] von Butandiol-1,4
N,N'-Hexamethylen-bis[β-(3-(benztriazole-2-yl)-4-butyroyloxy-5-methyl-phenyl)-propionsäureamid].

Die folgenden Beispiele erläutern die Herstellung solcher Verbindungen. Darin sind die Temperaturen in °C angegeben.

Herstellungsbeispiele

Beispiel A - Acetylierung

0,1 Mol 5-Chlor-2-[2-hydroxy-3,5-di(tert.-butyl)-phenyl]-benztriazol werden mit 100 ml Acetanhydrid vermischt und zum Rückfluss (140°) erhitzt. Nach Zugabe von 0,6 g 4-Dimethylaminopyridin erwärmt man weitere 7 Stunden zum Rückfluss. Eine Probe zeigt im Dünnschichtchromatogramm, dass kein Ausgangsmaterial mehr vorhanden ist. Das überschüssige Acetanhydrid wird im Vakuum abdestilliert und der braune Rückstand in Methylenchlorid gelöst. Die Lösung wird mit 5 %iger $Na_2CO_3$-Lösung und mit Wasser gewaschen, über $Na_2SO_4$ getrocknet und eingedampft. Der kristalline Rückstand wird aus Methanol umkristallisiert. Man erhält das 5-Chlor-2-[2-acetoxy-3,5-di(tert.-butyl)-phenyl]-benztriazol als weisse Kristalle, die bei 132-134° schmelzen (Verbindung Nr. 1).

In analoger Weise werden die folgenden Acetoxyverbindungen der allgemeinen Formel

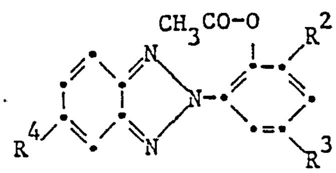

hergestellt

(Siehe Tabelle Seite 6 f.)

| Verbindung Nr. | $R^2$ | $R^3$ | $R^4$ | Charakterisierung |
|---|---|---|---|---|
| 2 | tert.-Butyl | $-CH_2CH_2COOCH_3$ | H | Smp. 67 - 69° (aus Ligroin) |
| 3 | tert.-Butyl | $-CH_2CH_2COOC_8H_{17}$ (Gemisch n- und iso-Octyl) | Cl | Oel<br>C ber. 65,8 % gef. 66,1 %<br>H ber. 7,4 % gef. 7,2 %<br>N ber. 7,9 % gef. 8,1 % |
| 4 | tert.-Butyl | $-CH_2CH_2COOCH_2CH_2OC_2H_5$ | H | viskoses Harz<br>C ber. 63,4 % gef. 63,4 %<br>H ber. 6,5 % gef. 6,5 %<br>N ber. 8,2 % gef. 8,2 % |
| 5 | tert.-Butyl | tert.-Butyl | H | Smp. 155 - 157° (aus Ligroin) |
| 6 | tert.-Pentyl (1,1-Dimethylpropyl) | tert.-Pentyl | H | Smp. 121 - 122° (aus Ligroin) |
| 7 | sec.-Butyl | tert.-Butyl | H | viskoses Harz<br>C ber. 72,3 % gef. 72,3 %<br>H ber. 7,4 % gef. 7,4 %<br>N ber. 11,5 % gef. 11,5 % |
| 8 | α-Dimethylbenzyl | α-Dimethylbenzyl | H | Smp. 180 - 182° (aus Toluol) |
| 9 | tert. Butyl | $-CH_2CH_2CONH(CH_2)_6NHCOCH_2CH_2-$ | H | Smp. 87-90°C |
| 10 | tert. Butyl | $-CH_2CH_2COOH$ | H | Smp. 134-136°C |

Beispiel B - Tosylierung

0,1 Mol 2-(2-Hydroxy-3-sec.-butyl-5-tert.-butyl-phenyl)-benztriazol werden in 200 ml Toluol gelöst. Dazu werden unter Rühren eine Lösung von 20 g NaOH in 20 g Wasser und 0,01 Mol Tetrabutylammoniumhydrogensulfat (als Phasentransferkatalysator) zugegeben, wobei sich die organische Phase intensiv gelb-orange färbt. Dann tropft man bei Raumtemperatur langsam eine Lösung von 0,11 Mol p-Toluolsulfochlorid in 50 ml Toluol zu. Die exotherme Reaktion wird hierbei durch Kühlung mit Eiswasser kompensiert. Anschliessend rührt man 3 Stunden bei Raumtemperatur wobei die Farbe der Toluollösung allmählich verschwindet. Eine Probe zeigt im Dünnschichtchromatogramm, dass kein Ausgangsmaterial mehr vorhanden ist. Das Reaktionsgemisch wird nun auf 1 l Eiswasser gegossen, die organische Phase wird abgetrennt, zweimal mit 300 ml Wasser gewaschen und über $Na_2SO_4$ getrocknet. Die Lösung wird im Vakuum eingedampft und der Rückstand aus Ethanol kristallisiert. Man erhält das 2-(2-Toluolsulfonyloxy-3-sec.-butyl-5-tert.-butyl-phenyl)-benztriazol als weisses Kristallisat, das bei 139-141 °C schmilzt (Verbindung Nr. 11).

In analoger Weise werden die folgenden Sulfonate der allgemeinen Formel

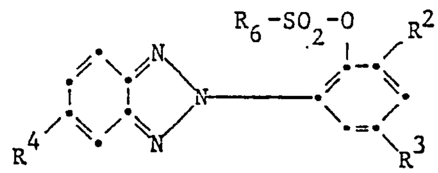

hergestellt.

(Siehe Tabelle Seite 8 f.)

| Verbindung Nr. | $R^2$ | $R^3$ | $R^4$ | $R^6$ | Charakterisierung |
|---|---|---|---|---|---|
| 12 | tert.-Butyl | tert.-Butyl | H | p-Tolyl | Smp. 187 - 189° (aus Ethanol) |
| 13 | tert.-Pentyl | tert.-Pentyl | H | p-Tolyl | Smp. 152 - 154° (aus Ethanol) |
| 14 | α-Dimethylbenzyl | α-Dimethylbenzyl | H | p-Tolyl | Smp. 152 - 154° (aus Isopropanol) |
| 15 | tert.-Butyl | $-CH_2CH_2COOCH_3$ | H | p-Tolyl | Smp. 123 - 125° (aus Methanol) |
| 16 | tert.-Butyl | $-CH_2CH_2COOC_8H_{17}$ (Gemisch n- und iso-Octyl) | Cl | | C ber. 63,8 % gef. 64,1 %<br>H ber. 6,6 % gef. 6,6 %<br>N ber. 6,6 % gef. 6,8 %<br>Cl ber. 5,5 % gef. 5,5 %<br>S ber. 5,0 % gef. 4,9 % |
| 17 | tert.-Pentyl | tert.-Pentyl | H | p-Dodecyl-phenyl | C ber. 72,8% gef. 72,7%<br>H ber. 8,7% gef. 8,7%<br>N ber. 6,4% gef. 6,5% |
| 18 | tert.-Butyl | tert.-Butyl | H | p-Dodecyl-phenyl | C ber. 72,3% gef. 72,0%<br>H ber. 8,5% gef. 8,5%<br>N ber. 6,7% gef. 6,8% |
| 19 | tert.-Butyl | tert.-Butyl | H | Methyl | Smp. 149-151° |
| 20 | tert.-Pentyl | tert.-Pentyl | H | Methyl | Smp. 88-90 |
| 21 | tert.-Butyl | $-CH_2CH_2COOCH_3$ | H | Methyl | Smp. 142-144° |
| 22 | α-Dimethylbenzyl | α-Dimethylbenzyl | H | Methyl | Smp. 176-178° |
| 23 | Sec. Butyl | tert.Butyl | H | p-Tolyl | Smp: 139-141°C |

Analog wird das Bis-tosylat der folgenden Formel hergestellt :

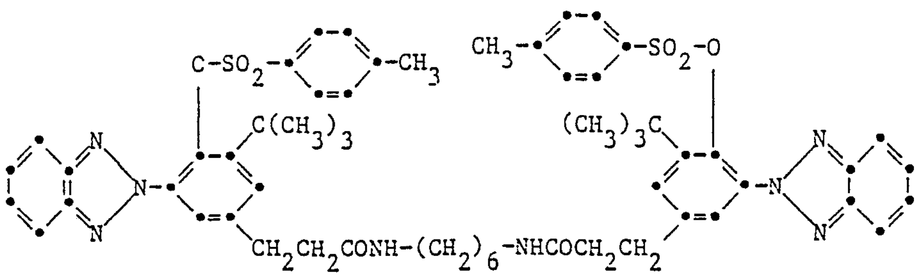

(Verbindung Nr. 24) Smp. : 215-217 °C

Beispiel C - Veresterung mit Säurechlorid

Eine Lösung von 0,08 Mol 2-[2-Hydroxy-3,5-di(tert.-butyl)-phenyl]-benztriazol in 150 ml Methylenchlorid wird mit 0,12 Mol Triethylamin versetzt. In diese Lösung tropft man eine Lösung von 0,08 Mol 2-Naphthylacetylchlorid in 50 ml Methylenchlorid langsam bei 10-15° unter Rühren zu. Dann lässt man die Temperatur auf 20-25° ansteigen und rührt 6 Stunden bei dieser Temperatur. Das ausgefallene Triethylammoniumchlorid wird abfiltriert und das Filtrat mit Wasser gewaschen, über $Na_2SO_4$ getrocknet und eingedampft. Der kristalline, rötlich gefärbte Rückstand wird aus Hexan umkristallisiert, wobei man das 2-[2-(1-Naphthylacetoxy)-3,5-di(tert.-butyl)-phenyl]-benztriazol als weisse Kristalle erhält, die bei 150-151° schmelzen (Verbindung Nr. 25).

In analoger Weise wurden die folgenden Phenolester der allgemeinen Formel

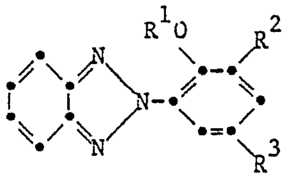

hergestellt :

(Siehe Tabelle Seite 10 f.)

| Verbindung Nr. | $R^1$ | $R^2$ | $R^3$ | Charakterisierung |
|---|---|---|---|---|
| 26 | 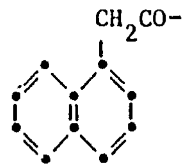 | sec.-Butyl | tert.-Butyl | Smp. 126-128° |
| 27 | $-CO-CH_2-$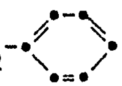 | tert.-Butyl | tert.-Butyl | Smp. 178-180° |
| 28 | $-CO-CH_2-$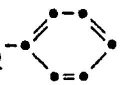 | tert.-Pentyl | tert.-Pentyl | Smp. 74-76° |
| 29 | $-CO-CH_2-$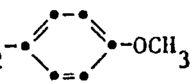 | tert.-Butyl | tert.-Butyl | Smp. 114-116° |
| 30 | $-CO-CH_2-$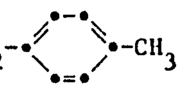 | tert.-Butyl | tert.-Butyl | Smp. 106-108° |
| 31 | $-CO-COOC_2H_5$ | tert.-Butyl | $-(CH_2)_2COOCH_3$ | Smp. 82-84° |
| 32 | $-CO-COOC_2H_5$ | tert.-Butyl | tert.-Butyl | Smp. 112-114° |
| 33 | $-CO-COOC_2H_5$ | tert.-Pentyl | tert.-Pentyl | Smp. 81-83° |
| 34 | $-CO-COOC_2H_5$ | α-Dimethyl-benzyl | α-Dimethylbenzyl | Smp. 106-108° |
| 35 | $-CO-CH=CH_2$ | tert.-Butyl | tert.Butyl | Smp. 104-106° |
| 36 | $-CO-CH=CH_2$ | tert.-Pentyl | tert.-Pentyl | Smp. 101-103° |
| 37 | $-CO-CH=CH_2$ | α-Dimethyl-benzyl | α-Dimethylbenzyl | Smp. 128-130° |
| 38 | $-CO-CH=CH_2$ | tert.-Butyl | $-CH_2CH_2COOCH_3$ | Smp. 84-86° |

Beispiel D - Veresterung mit Säurechloriden durch Phasentransferkatalyse

0,1 Mol 2-[2-Hydroxy-3,5-di(tert.butyl) phenyl]-benztriazol wird in 150-200 ml Toluol gelöst. Dazu gibt man 20 g NaOH in 20 ml Wasser gelöst (50 %ige NaOH-Lösung) und 0,01 Mol Tetrabutylammoniumhydrogensulfat (PTC). Es entsteht sofort eine orangefarbige Emulsion. Diese wird auf 40° erwärmt. Bei dieser Temperatur lässt man langsam eine Lösung von 0,11 Mol Chlorameisensäurebutylester in ca. 30 ml Toluol gelöst hinzutropfen. Die Reaktion verläuft exotherm und wird ab 45-50° mit Eis/Wasser gekühlt. Am Ende des Zutropfens wird die Reaktionslösung hellgelb. Man lässt noch ca. 2 Stunden ausrühren, danach kann im Dünnschichtchromatogramm kein Ausgangsprodukt mehr festgestellt werden. Man kühlt die Reaktionslösung auf Raumtemperatur ab und fügt ca. 200 ml Wasser hinzu. Danach trennt man die organische Phase ab und wäscht noch zweimal mit je 100 ml Wasser. Die Toluollösung wird mit Natriumsulfat getrocknet und danach am Rotationsverdampfer eingedampft. Der hellgelbe Rückstand wird aus Ethanol umkristallisiert. Man erhält 2-[2-(Butoxycarbonyloxy)-3,5-di(tert.-butyl)-phenyl]-benztriazol (Verbindung Nr. 39) als weisses Kristallisat, das bei 116-118 °C schmilzt.

In analoger Weise werden durch Variation des Säurechlorids folgende phenolische Ester hergestellt :

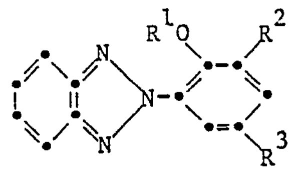

(Siehe Tabelle Seite 12 ff.)

| Verbindung Nr. | $R^1$ | $R^2$ | $R^3$ | Charakterisierung |
|---|---|---|---|---|
| 40 | $-COOC_4H_9$ | tert.-Pentyl | tert.-Pentyl | C ber. 71,0% gef. 70,9%<br>H ber. 8,4% gef. 8,3%<br>N ber. 9,5% gef. 9,3% |
| 41 | $-COOC_4H_9$ | α-Dimethyl-benzyl | α-Dimethylbenzyl | C ber. 76,7% gef. 76,9%<br>H ber. 6,8% gef. 6,7%<br>N ber. 7,6% gef. 7,7% |
| 42 | $-COOC_4H_9$ | tert.Butyl | $-CH_2CH_2COOCH_3$ | C ber. 66,2% gef. 66,2%<br>H ber. 7,0% gef. 6,9%<br>N ber. 9,5% gef. 9,7% |
| 43 | $-CO(CH_2)_{10}CH_3$ | tert.-Pentyl | tert.-Pentyl | Smp. 51-54° |
| 44 | $-CO(CH_2)_{10}CH_3$ | α-Dimethyl-benzyl | α-Dimethylbenzyl | C ber. 80,0% gef. 80,1%<br>H ber. 8,1% gef. 8,2%<br>N ber. 6,6% gef. 6,7% |
| 45 | $-CO(CH_2)_6CH_3$ | tert.-Butyl | tert.-Butyl | C ber. 74,7% gef. 74,9%<br>H ber. 8,7% gef. 8,7%<br>N ber. 9,3% gef. 9,3% |
| 46 | $-CO(CH_2)_6CH_3$ | tert.-Pentyl | tert.-Pentyl | C ber. 75,4% gef. 75,4%<br>H ber. 9,0% gef. 9,0%<br>N ber. 8,8% gef. 8,8% |

| Verbindung Nr. | $R^1$ | $R^2$ | $R^3$ | Charakterisierung |
|---|---|---|---|---|
| 47 | $-CO(CH_2)_6CH_3$ | α-Dimethyl-benzyl | α-Dimethylbenzyl | C ber. 79,5% gef. 79,5%<br>H ber. 7,5% gef. 7,6%<br>N ber. 7,3% gef. 7,4% |
| 48 | $-CO(CH_2)_6CH_3$ | tert.-Butyl | $-CH_2CH_2COOCH_3$ | C ber. 70,1% gef. 70,2%<br>H ber. 7,7% gef. 7,7%<br>N ber. 8,7% gef. 8,8% |
| 49 | $-PO(OC_2H_5)_2$ | tert.-Butyl | tert.-Butyl | Smp. 118-120° |
| 50 | $-PO(OC_2H_5)_2$ | tert.-Pentyl | tert.-Pentyl | Smp. 93-95° |
| 51 | $-PO(OC_2H_5)_2$ | α-Dimethyl-benzyl | α-Dimethylbenzyl | Smp. 124-126° |
| 52 | -CO- 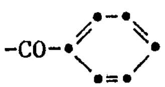 | tert.-Butyl | tert.-Butyl | Smp. 154-156° |
| 53 | -CO- 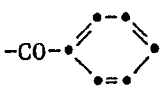 | tert.-Pentyl | tert.-Pentyl | Smp. 118-120° |
| 54 | -CO- 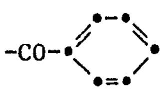 | α-Dimethyl-benzyl | α-Dimethylbenzyl | Smp. 143-145° |
| 55 | -CO- 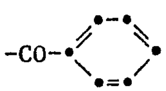 | tert.-Butyl | $-CH_2CH_2COOCH_3$ | Smp. 122-124° |
| 56 | -CO- 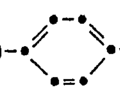 -CO- 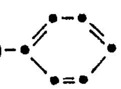 | tert.-Butyl | tert.-Butyl | Smp. 159-161° |

| Verbindung Nr. | $R_1$ | $R_2$ | $R_3$ | Charakterisierung |
|---|---|---|---|---|
| 57 | 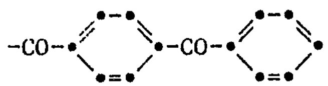 | tert.-Pentyl | tert.-Pentyl | Smp. 82-85° |
| 58 | 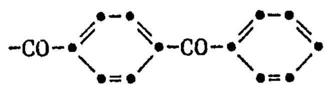 | α-Dimethylbenzyl | α-Dimethylbenzyl | Smp. 172-174° |
| 59 | 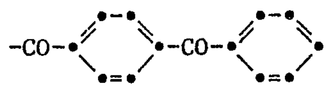 | tert.-Butyl | $-CH_2CH_2COOCH_3$ | C ber. 72,7% gef. 72,3%<br>H ber. 5,5% gef. 5,6%<br>N ber. 7,4% gef. 7,6% |
| 60 | 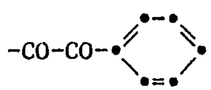 | tert.-Butyl | tert.-Butyl | Smp. 137-138° |

Die erfindungsgemässen Ueberzugsmittel können pigmentiert oder unpigmentiert sein. Als Hauptbestandteil enthalten sie ein Bindemittel, das ein zunächst lösliches Harz ist, das nach der Applikation in einen unlöslichen Zustand übergeführt wird. Die Härtung kann durch Erhitzen oder durch chemische Reaktion erfolgen. Beispiele für solche Bindemittel sind Alkylharze, Acrylharze, Epoxydharze, Melaminharze, Harnstoffharze, Polyurethane, Polyester oder Phenolharze und deren Gemische.

Zur Applikation wird das Ueberzugsmittel meist als Lösung aufgebracht. Ueberzugsmittel mit wenig Lösungsmittel (high-solids) oder ohne Lösungsmittel (unter Zusatz von Reaktivverdünnern) spielen neuerdings eine zunehmende Rolle in der Anstrichtechnik. In all diesen Fällen kann der Zusatz eines Lichtschutzmittels von Interesse sein. Beispiele hierfür sind Decklacke bei Mehrschicht-Anstrichen, die die untere pigmentierte Schicht gegen Lichtschädigung schützen soll. Solche Anstriche finden vor allem im Fahrzeugbau Verwendung. Weitere Beispiele sind UV-Schutzlacke für Möbel oder für Plastikfolien. Von besonderer Bedeutung ist die Erfindung für Ueberzugsmittel, die bei ihrer Applikation mit Kupfer oder Kupferlegierungen in Kontakt kommen.

Je nach ihrem Verwendungszweck können die Ueberzugsmittel ausser Pigmenten und Lösungsmitteln noch andere Additive enthalten, wie z. B. Verlaufhilfsmittel, Thixotropiemittel, Netzmittel, Metalldesaktivatoren oder Antioxydantien. Sie können fernerhin ausser dem erfindungsgemässen Kryptolichtschutzmittel noch ein Lichtschutzmittel aus der Klasse der sterisch gehinderten Amine enthalten, da diese ebenfalls nicht im UV-Bereich von 330-400 nm absorbieren.

Die erfindungsgemässen Ueberzugsmittel können auf beliebige Substrate aufgebracht werden, z. B. auf Metall-, Glas-, Keramik-, Holz-, Papier- oder Plastik-Oberflächen. Der Auftrag geschieht nach den für Ueberzugsmittel üblichen Methoden, z. B. durch Streichen, Tauchen, Besprühen oder durch elektrostatische Prozesse. Die Aktivierung des Lichtschutzmittels erfolgt in dem Ausmass, in dem kurzwelliges Licht auf den Ueberzug einwirkt. Bei schwacher Bestrahlung wird also eine langsame Aktivierung erfolgen, während bei intensiver Bestrahlung eine rasche Aktivierung erfolgt. Man kann das Lichtschutzmittel auch künstlich aktivieren, indem man den Ueberzug vor seiner Verwendung mit UV-Licht bestrahlt. Dies kann vor oder nach der Härtung des Ueberzuges geschehen. Die Härtung des Ueberzuges erfolgt entsprechend der Art des verwendeten Bindemittels, beispielsweise durch Erwärmen oder durch oxydative Härtung.

Die folgenden Beispiele erläutern die erfindungsgemässe Verwendung von Krypto-Lichtschutzmitteln. Darin bedeutet % Gewichts-Prozente soweit nicht anders angegeben.

Beispiel 1 : Stabilisierung eines 2-Schicht-Metalleffekt-Lackes

Aluminiumbleche von 0,5 mm Stärke werden mit einem Aluminium-pigmentierten Grundlack auf Basis Polyester/Celluloseacetobutyrat/Melaminharz beschichtet. Auf den nassen Grundlack wird ein Klarlack der folgenden Zusammensetzung aufgespritzt :

58,3 Teile Viacryl® VC 373 (Acrylharz der Fa. Vianova, Wien)
27,3 Teile Maprenal® MF 590 (Melaminharz der Fa. Höchst AG, Frankfurt)
1,0 Teil 1 %ige Lösung eines Silikonöles in Xylol
4,0 Teile Solvesso® 150 (aromatisches Lösungsmittelgemisch)
5,4 Teile Xylol
4,0 Teile Ethylglykolacetat.

Dazu kommen 0,9 Teile des in der Tabelle 1 angegebenen Kryptolichtschutzmittels, entsprechend 2 % bezogen auf Bindemittel. Dieser Klarlack wird auf eine Viskosität von 21 sec/DIN-Becher 4 eingestellt. Er wird in einer Trocken-Schichtdicke von 40 μm aufgetragen und bei 130 °C 30 Minuten eingebrannt.

Die Proben werden einerseits in einem QUV-Schnellbewitterungsgerät der Fa. Q-Panel und andererseits in einem Xenotest® 1200 der Fa. Heraeus bis zu 5200 Stunden bewittert. Nach jeweils 800 Stunden wird der 20°-Glanz gemäss DIN 67530 gemessen und die Oberfläche der Proben unter dem Stereomikroskop auf Rissbildung untersucht. Als Vergleich wurde a) eine unstabilisierte Probe bewittert b) ein nicht-blockierter UV-Absorber der Formel

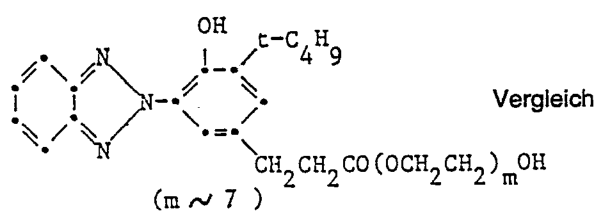

(m ∼ 7 )

Vergleich A

verwendet. Die Ergebnisse sind in den Tabellen 1 und 2 aufgeführt.

(Siehe Tabellen Seite 16 f.)

Tabelle 1-QUV-2-Bewitterung

| Lichtschutzmittel | 20°-Glanz nach 0 | 1600 | 3200 | 4800 h | Rissbildung bemerkbar nach |
|---|---|---|---|---|---|
| Keines | 91 | 42 | - | - | 2800 h |
| Verbindung Nr. 2 | 91 | 79 | 76 | 26 | 5200 h |
| Vergleich A | 90 | 81 | 69 | 29 | 5200 h |

Tabelle 2-Xenotest® 1200-Bewitterung

| Lichtschutzmittel | 20°-Glanz nach 0 | 1600 | 3200 | 4800 h | Rissbildung bemerkbar nach |
|---|---|---|---|---|---|
| Ohne | 91 | 38 | 31 | - | 3600 h |
| Verbindung Nr. 2 | 91 | 68 | 54 | 31 | 5200 h |
| Vergleich A | 90 | 61 | 44 | 28 | 5200 h |

Wie man daraus ersieht ist die Schutzwirkung des Kryptolichtschutzmittels etwa gleich der des unblockierten Lichtschutzmittels.

Beispiel 2 : Verfärbung durch Kontakt mit Kupfer

Der in Beispiel 1 beschriebene Klarlack wird vor der Applikation 48 h mit einem Kupferblech in Kontakt gebracht. Dann wird der Lack in einer Schichtdicke von 40 µm auf Bleche, die mit einem $TiO_2$-pigmentierten Weisslack auf Polyesterharz-Basis grundiert sind, aufgetragen. Die Proben werden bei 130° 30 Minuten eingebrannt.

Die Verfärbung der Proben wird einerseits nach dem Yellowness-Index (YI), andererseits nach DIN 6174 (als ΔE) gemessen.

Als Vergleich wird der handelsübliche UV-Absorber 2-[2-Hydroxy-3,5-di-(1,1-dimethylbenzyl)-phenyl]-benztriazol = Vergleich B verwendet.

Tabelle 3

| Lichtschutzmittel | YI | ΔE |
|---|---|---|
| Keines | 3,4 | 2,1 |
| 1 % Verbindung Nr. 2 | 3,1 | 2,0 |
| 1 % Vergleich B | 5,8 | 3,2 |

Man ersieht daraus, dass das freie Hydroxyphenyl-benztriazol eine Vergilbung bewirkt, das Krypto-Lichtschutzmittel jedoch nicht.

Beispiel 3 : Lagerstabilität der Kryptolichtschutzmittel

Methanol-Lösungen von $5.10^{-5}$ Mol/l Benzotriazolderivat werden 14 Tage im Dunkeln gelagert. Das UV-Spektrum der Lösungen wird vor und nach der Lagerung gemessen. Hierbei zeigen die Lösungen der Verbindungen Nr. 2, 5, 6, 7, 8, 11, 12, 13, 14, 15, 17 und 18 keine Veränderung des Spektrums.

Aehnliche blockierte Benztriazolderivate, die in ortho-Position zur blockierten OH-Gruppe unsubstituiert sind, zeigen nach der Lagerung eine Veränderung des Spektrums, die darauf hinweist, dass während der Dunkellagerung eine teilweise Deblockierung eingetreten ist.

Beispiel 4

Ein Zweikomponenten-Einbrennlack auf Acrylharzbasis wird aus folgenden Komponenten bereitet :

Teil A

72,8 g eines aminfunktionellen Acrylharzes (Setalux® 83-03 BX 55 der Fa. Synthese, Niederlande)
0,9 g eines Verlaufshilfsmittels auf Basis Silikonöl (Baysilon®-Oel, Fa. Bayer AG)
9,0 g Xylol
82,7 g

Teil B

17,3 g eines epoxyfunktionellen Acrylharzes (Setalux® 83-04 55 70 der Fa. Synthese, NL)

Die Teile A und B werden vereinigt und mit einer Lösung des in Tabelle 4 angegebenen Lichtschutzmittels in Xylol versetzt. Dieser Lack wird aufgespritzt auf ein Blech, das mit einem silbermetallic Einbrennlack auf Basis Polyester/Celluloseacetobutyrat/Melaminharz grundiert ist. Nach einstündigem Ablüften bei Raumtemperatur werden die Proben 30 Minuten bei 135 °C eingebrannt. Die Schichtdicke des Decklackes beträgt ca. 40 µm.

Die beim Einbrennen eingetrenene Verfärbung wird gemäss DIN 6174 als Farbiondifferenz ΔE gemessen.

Es werden jeweils die freien Hydroxybenztriazole und deren o-Acyl-verbindungen verglichen.

Tabelle 4

| Lichtschutzmittel | ΔE |
|---|---|
| Keines | 0,7 |
| 2 % Verbindung Nr. 8 | 1,0 |
| 2 % Vergleich B | 12,8 |
| 2 % Verbindung Nr. 6 | 0,7 |
| 2 % Vergleich C | 4,4 |

Vergleich B = 2-[2-Hydroxy-3,5-di(1,1-dimethylbenzyl)-phenyl]-benztriazol
Vergleich C = 2-[2-Hydroxy-3,5-di(tert.pentyl)-phenyl]-benztriazol

Beispiel 5 : Verfärbung bei Bewitterung

Der in Beispiel 1 beschriebene Klarlack wird in einer Trockenfilmstärke von 40 µm auf weiss grundierte Bleche aufgebracht. Die Proben werden 30 Minuten bei 130° eingebrannt und 3 Monate in Florida bewittert. Vor und nach der Bewitterung wird der Weissgrad nach DIN 6174 gemessen und daraus der Farbabstand ΔE ermittelt. Die Resultate sind in Tabelle 5 aufgeführt.

Tabelle 5

| Lichtschutzmittel | ΔE |
|---|---|
| Keines | 3,2 |
| 2 % Verbindung Nr. 6 | 1,0 |
| 2 % Verbindung Nr. 13 | 0,8 |
| 2 % Verbindung Nr. 20 | 0,6 |
| 2 % Verbindung Nr. 46 | 1,0 |

Beispiel 6 : Kombination mit UV-deblockierbarem Katalysator

Ein säurehärtbarer Acrylharz-Melamin-Klarlack wird bereitet aus :

57,3 Teilen Acryloid® AT 410 (Acrylharz der Fa. Rohm & Haas Corp., USA) (75 % Feststoff)
18,0 Teilen Cymel® 301 (Melaminharz der Fa. Amer. Cyanamid)
10,0 Teilen Butylacetat
1,8 Teilen Celluloseacetobutyrat CAB 551 (Eastman Chem. Corp.)
2,6 Teilen Verlaufhilfsmittel (Modaflow®, Monsanto Corp.)
10,3 Teilen Butanol

Der Feststoffgehalt des Lackes beträgt 65 %. Der Lack wird in einer Trockenfilmstärke von 40 μm auf Glasplatten aufgerakelt. Die Proben werden 10 Sekunden mit einer 160 W-UV-Lampe belichtet und dann 30 Minuten bei 120° eingebrannt. Als UV-aktivierbarer Härtungskatalysator wird 1 % Benzoin-p-toluolsulfonat verwendet, welches bei UV-Belichtung p-Toluolsulfonsäure abspaltet. Ein solcher blockierter Säurekatalysator hat den Vorteil, dass er im Dunkeln keine Härtung bewirkt. Er hat den Nachteil, dass in Gegenwart von UV-Absorber-Lichtschutzmitteln die Härtung des Lackes unvollständig ist. Verwendet man jedoch als Lichtschutzmittel die erfindungsgemässen O-acylierten Benztriazole, so wird die Aktivität des Härtungskatalysators nicht gehindert, wie die folgende Tabelle 6 zeigt. Hierbei wird die Härtung des Lackes durch Messung der Pendelhärte nach DIN 53157 beurteilt. Die Proben werden anschliessend im Xenon-Weatherometer belichtet und die prozentuelle Glanzerhaltung ermittelt.

Tabelle 6

| Lichtschutzmittel | Glanzerhaltung in % nach Bewitterung von | | | Pendelhärte |
|---|---|---|---|---|
| | 1000 h | 2000 h | 3000 h | (sec) |
| Keines | 83 | 5 | - | 204 |
| 1 % Vergleich C *) | 91 | 82 | 4 | 78 |
| 1 % Verbindung Nr. 2 | 70 | 56 | 2 | 154 |
| 1 % Verbindung Nr. 5 | 75 | 77 | 6 | 155 |
| 1 % Verbindung Nr. 5 + 1 % HALS **) | 102 | 63 | 23 | 122 |
| 1 % Verbindung Nr. 6 | 98 | 78 | 5 | 158 |

*) Vergleich C = 2-(2-Hydroxy-3,5-di-tert · pentyl-phenyl)-benztriazol (Tinuvin® 328)
**) HALS = 8-Acetyl-3-dodecyl-7,7,9,9-tetramethyl-1,3,8-triaza-spiro [4,5] decandion-2,4 (Tinuvin® 440)

Man sieht daraus, dass das freie Benztriazol (Vergleich C) zwar einen guten Lichtschutz bewirkt, jedoch die Härtung des Lackes stark vermindert. Mit den erfindungsgemässen Lichtschutzmitteln erreicht man sowohl einen guten Lichtschutz als auch eine befriedigende Härtung. Der Lichtschutz lässt sich durch Zusatz eines Lichtschutzmittels vom Typ der sterisch gehinderten Amine (HALS) steigern.

## Patentansprüche

1. Ueberzugsmittel, enthaltend als Kryptolichtschutzmittel eine Verbindung der Formel I oder II,

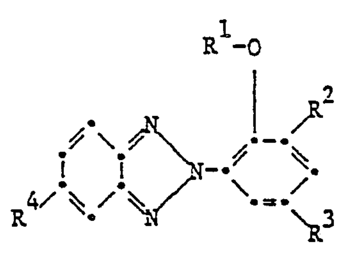

(I)

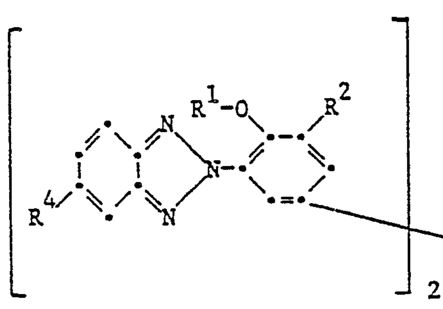

(II)

worin

$R^1$ eine Acylgruppe der Formel $—CO—R^5$, eine Sulfonylgruppe der Formel $—SO_2—R^6$, eine Phosphorylgruppe der Formel $—P(O)_r(R^{14})$ $(R^{15})$ bedeutet,

$R^2$ $C_1$-$C_{12}$-Alkyl, $C_5$-$C_{12}$-Cycloalkyl, Phenyl, $C_7$-$C_9$-Phenylalkyl, $C_3$-$C_5$-Alkenyl oder Halogen bedeutet,

$R_3$ in Formel I $C_1$-$C_{12}$-Alkyl, $C_5$-$C_{12}$-Cycloalkyl, Phenyl, $C_7$-$C_9$-Phenylalkyl, Halogen oder eine Gruppe der Formel $—(CH_2)_n—COOR^9$ oder $—(CH_2)_nCON(R^{10})$ $(R^{11})$ bedeutet und in Formel II einen zweiwertigen Rest der Formeln $—(CH_2)_n—CO—O—(CH_2)_q—O—CO—(CH_2)_n—$, $—(CH_2)_n—CO—O—(CH_2CH_2O)_p—CO—(CH_2)_n—$, $—(CH_2)_n—CO—NH—R^{12}—NH—CO—(CH_2)_n—$ oder

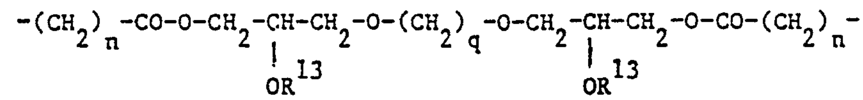

bedeutet,

$R^4$ Wasserstoff, Halogen, $C_1$-$C_8$-Alkyl, $C_7$-$C_9$-Phenylalkyl, $C_1$-$C_8$-Alkoxy oder $C_2$-$C_8$-Alkoxycarbonyl bedeutet,

$R^5$ $C_1$-$C_{18}$-Alkyl, $C_2$-$C_{18}$-Alkenyl, $—CH_2—CO—CH_3$, Phenyl, durch $C_1$-$C_{12}$-Alkyl, $C_1$-$C_4$-Alkoxy oder Benzoyl substituiertes Phenyl, $C_7$-$C_{12}$-Arylalkyl oder $C_1$-$C_{12}$-Alkoxy bedeutet,

$R^6$ $C_1$-$C_{12}$-Alkyl, $C_6$-$C_{10}$-Aryl oder $C_7$-$C_{18}$-Alkylaryl bedeutet,

$R^9$ Wasserstoff $C_1$-$C_{12}$-Alkyl oder eine Gruppe der Formel $—(CH_2CH_2O)_p—R^1$ bedeutet,

$R^{10}$ und $R^{11}$ unabhängig voneinander Wasserstoff, $C_1$-$C_{12}$-Alkyl, das durch O oder N unterbrochen sein kann, $C_5$-$C_{12}$-Cycloalkyl, $C_7$-$C_9$-Phenylalkyl, $C_3$-$C_5$-Alkenyl oder Phenyl oder einen 2,2,6,6-Tetramethyl-4-piperidinyl-Rest bedeuten, oder $R^{10}$ und $R^{11}$ zusammen $C_4$-$C_6$-Alkylen, -Oxaalkylen oder -Azaalkylen bedeuten,

$R^{12}$ $C_1$-$C_{12}$-Alkylen, das durch 1-3 O-Atome unterbrochen sein kann, bedeutet,

$R^{13}$ $C_1$-$C_{12}$-Alkyl oder $C_6$-$C_{10}$-Aryl bedeutet,

$R^{14}$ und $R^{15}$ unabhängig voneinander $C_1$-$C_{12}$-Alkoxy, Phenoxy, $C_1$-$C_{12}$-Alkyl, Cyclohexyl, Benzyl, Phenyl oder Tolyl bedeuten,

n 1 oder 2,

p eine Zahl von 1 bis 10,

q eine Zahl von 2 bis 12 und r 0 oder 1 ist.

2. Ueberzugsmittel gemäss Anspruch 1, enthaltend eine Verbindung der Formel I, worin

$R^1$ eine Gruppe der Formel $—CO—R^5$, $—SO_2—R^6$, oder $—P(O)$ $(R^{14})$ $(R^{15})$,

$R^2$ $C_1$-$C_{12}$-Alkyl, Cyclohexyl oder $C_7$-$C_9$-Phenylalkyl,

$R^3$ $C_1$-$C_{12}$-Alkyl, Cyclohexyl, $C_7$-$C_9$-Phenylalkyl oder eine Gruppe $—CH_2CH_2COOR^9$

$R^4$ Wasserstoff, Methyl oder Chlor,

$R^5$ $C_1$-$C_{12}$-Alkyl, $C_2$-$C_4$-Alkenyl, Phenyl, Benzyl oder Naphthylmethyl

$R^6$ Methyl, Phenyl oder $C_7$-$C_{18}$-Alkylphenyl und

$R^9$ $C_1$-$C_{12}$-Alkyl und

$R^{14}$ und $R^{15}$ unabhängig voneinander $C_1$-$C_4$-Alkoxy, Methyl oder Phenyl bedeuten.

3. Ueberzugsmittel gemäss Anspruch 2, wobei $R^1$ eine Gruppe der Formel $—CO—R^5$ oder $—SO_2—R^6$ ist und $R^5$ und $R^6$ die in Anspruch 2 gegebenen Bedeutungen haben.

4. Ueberzugsmittel gemäss Anspruch 3, wobei $R^2$ $C_1$-$C_8$-Alkyl oder α-Dimethylbenzyl ist, $R^3$ $C_1$-$C_8$-Alkyl, α-Dimethylbenzyl oder eine Gruppe $—CH_2CH_2COOR^9$ ist, $R^4$ Wasserstoff oder Chlor ist und $R^1$ und $R^9$ die in Anspruch 3 gegebenen Bedeutungen haben.

5. Ueberzugsmittel gemäss Anspruch 1, enthaltend eine Verbindung der Formel I, worin $R^1$ eine Gruppe $—CO—R^5$ oder $—SO_2—R^6$ ist, $R^2$ $C_1$-$C_8$-Alkyl oder α-Dimethylbenzyl ist, $R^3$ $C_1$-$C_8$-Alkyl, α-Dimethylbenzyl oder $—CH_2CH_2COOR^9$ ist, $R^4$ Wasserstoff oder Chlor ist, $R^5$ $C_1$-$C_{12}$-Alkyl, $C_2$-$C_4$-Alkenyl, Phenyl, Benzyl oder Naphthylmethyl bedeutet, $R^6$ Methyl, Phenyl oder $C_7$-$C_{18}$-Alkylphenyl bedeutet und $R^9$ $C_1$-$C_{12}$-Alkyl ist.

6. Ueberzugsmittel gemäss Anspruch 1, enthaltend eine Verbindung der Formel II, worin $R^1$ eine Gruppe der Formel $—CO—R^5$ oder $—SO_2—R^6$ ist, $R^2$ $C_1$-$C_8$-Alkyl oder α-Dimethylbenzyl bedeutet, $R^3$ eine Gruppe der Formel $—CH_2CH_2COO—(CH_2)_q—OCOCH_2CH_2—$ oder $—CH_2CH_2CONH—R^{12}—NHCOCH_2CH_2—$ bedeutet, $R^4$ Wasserstoff oder Chlor ist, $R^5$ $C_1$-$C_{12}$-Alkyl, $C_2$-$C_4$-Alkenyl, Phenyl, Benzyl oder Naphthylmethyl bedeutet, $R^6$ Methyl, Phenyl oder $C_7$-$C_{18}$-Alkylphenyl bedeutet, $R^{12}$ $C_2$-$C_8$-Alkylen und q eine Zahl von 2 bis 8 ist.

7. Ueberzugsmittel gemäss Anspruch 1, enthaltend Bindemittel aus der Reihe Alkydharze, Acrylharze, Epoxidharze, Melaminharze, Harnstoffharze, Polyurethane, Polyester und Phenolharze und deren Gemische.

8. Ueberzugsmittel gemäss Anspruch 1, enthaltend als Bindemittel ein oxydativ trocknendes Harz oder Harzgemisch und als Härtungskatalysator eine organische Metallverbindung.

9. Anwendung des Ueberzugsmittels gemäss Anspruch 1 auf Kupfer oder einer Kupferlegierung.

10. Verbindungen der Formel I oder II

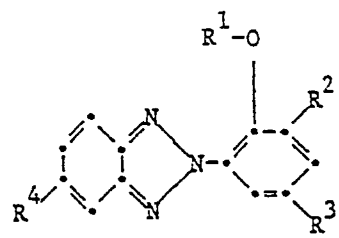

(I)

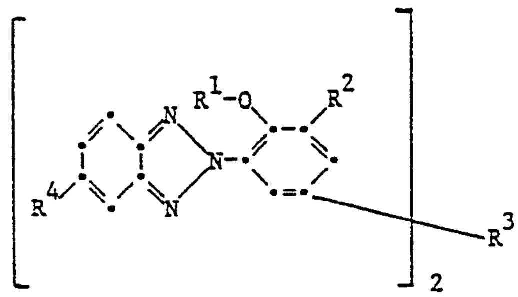

(II)

worin

$R^1$ eine Acylgruppe der Formel —CO—$R^5$ oder eine Sulfonylgruppe der Formel —$SO_2$—$R^6$ oder eine Phosphorylgruppe der Formel —$P(O)_r$ ($R^{14}$) ($R^{15}$) bedeutet,

$R^2$ $C_1$-$C_{12}$-Alkyl, $C_5$-$C_{12}$-Cycloalkyl, Phenyl, $C_7$-$C_9$-Phenylalkyl, $C_3$-$C_5$-Alkenyl oder Halogen bedeutet,

$R^3$ in Formel I eine Gruppe der Formel —$(CH_2)_n$—$COOR^9$ oder —$(CH_2)_n$—CON($R^{10}$) ($R^{11}$) bedeutet und in Formel II eine zweiwertige Gruppe der Formeln —$(CH_2)_n$—CO—O—$(CH_2)_q$—O—CO—$(CH_2)_n$—, —$(CH_2)_n$—CO—O—$(CH_2CH_2O)_p$—CO—$(CH_2)_n$—, —$(CH_2)_n$—CO—NH—$R^{12}$—NH—CO—$(CH_2)_n$— oder

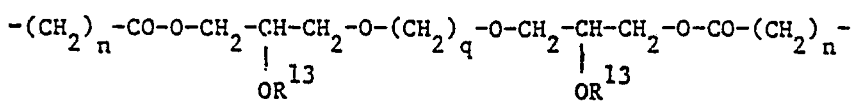

bedeutet,

$R^4$ Wasserstoff, Halogen, $C_1$-$C_8$-Alkyl, $C_7$-$C_9$-Phenylalkyl, $C_1$-$C_8$-Alkoxy oder $C_2$-$C_8$-Alkoxycarbonyl bedeutet,

$R^5$ $C_1$-$C_{18}$-Alkyl, $C_2$-$C_{18}$-Alkenyl, —$CH_2$—CO—$CH_3$, Phenyl, durch $C_1$-$C_{12}$-Alkyl, $C_1$-$C_4$-Alkoxy oder Benzoyl substituiertes Phenyl, $C_7$-$C_{12}$-Arylalkyl oder $C_1$-$C_{12}$-Alkoxy bedeutet,

$R^6$ $C_1$-$C_{12}$-Alkyl, $C_6$-$C_{10}$-Aryl oder $C_7$-$C_{18}$-Alkylaryl bedeutet,

$R^7$ $C_1$-$C_{10}$-Alkylen oder Phenylen bedeutet,

$R^9$ Wasserstoff $C_1$-$C_{12}$-Alkyl oder eine Gruppe der Formel —$(CH_2CH_2O)_p$—$R^1$ bedeutet,

$R^{10}$ und $R^{11}$ unabhängig voneinander Wasserstoff, $C_1$-$C_{12}$-Alkyl, das durch O oder N unterbrochen sein kann, $C_5$-$C_{12}$-Cyloalkyl, $C_7$-$C_9$-Phenylalkyl, $C_3$-$C_5$-Alkenyl, Phenyl, oder einen 2,2,6,6-Tetramethyl-4-piperidinyl-Rest bedeuten, oder $R^{10}$ und $R^{11}$ zusammen $C_4$-$C_6$-Alkylen, -Oxaalkylen oder -Azaalkylen bedeuten,

$R^{12}$ $C_1$-$C_{12}$-Alkylen, das durch 1-3 O-Atome unterbrochen sein kann, bedeutet,

$R^{13}$ $C_1$-$C_{12}$-Alkyl oder $C_6$-$C_{10}$-Aryl bedeutet,

$R^{14}$ und $R^{15}$ unabhängig voneinander $C_1$-$C_{12}$-Alkoxy, Phenoxy, $C_1$-$C_{12}$-Alkyl, Cyclohexyl, Benzyl, Phenyl oder Tolyl bedeuten,

n 1 oder 2,

p eine Zahl von 1 bis 10,

q eine Zahl von 2 bis 12 und

r 0 oder 1 ist.

11. Verbindungen der Formel I gemäss Anspruch 10, worin

$R^1$ eine Gruppe der Formel —CO—$R^5$ oder —$SO_2$—$R^6$ ist,

$R^2$ $C_1$-$C_{12}$-Alkyl, Cyclohexyl oder $C_7$-$C_9$-Phenylalkyl bedeutet,

$R^3$ eine Gruppe —$CH_2CH_2COOR^9$ ist,

$R^4$ Wasserstoff, Methyl oder Chlor ist,

$R^5$ $C_1$-$C_{12}$-Alkyl, $C_2$-$C_4$-Alkenyl, Phenyl, Benzyl oder Naphthylmethyl bedeutet,

$R^6$ Methyl, Phenyl oder $C_7$-$C_{15}$-Alkylphenyl bedeutet.

12. Verbindungen der Formel I gemäss Anspruch 11, worin $R^1$ eine Gruppe —CO—$R^5$ oder —$SO_2$—$R^6$ ist, $R^2$ $C_1$-$C_8$-Alkyl oder α-Dimethylbenzyl ist, $R^4$ Wasserstoff oder Chlor ist und $R^3$, $R^5$ und $R^6$ die in Anspruch 11 gegebenen Bedeutungen haben.

13. Verbindungen der Formel II gemäss Anspruch 10, worin
$R^1$ eine Gruppe $—CO—R^5$ oder $—SO_2—R^6$ ist,
$R^2$ $C_1$-$C_8$-Alkyl oder α-Dimethylbenzyl ist,
$R^3$ eine Gruppe der Formel $—CH_2CH_2COO—(CH_2)_q—OCOCH_2CH_2—$ oder $—CH_2CH_2CONH—R^{12}—NHCOCH_2CH_2—$ bedeutet,
$R^4$ Wasserstoff oder Chlor ist,
$R^5$ $C_1$-$C_{12}$-Alkyl, $C_2$-$C_4$-Alkenyl, Phenyl, Benzyl oder Naphthylmethyl bedeutet,
$R^6$ Methyl, Phenyl oder $C_7$-$C_{18}$-Alkylphenyl bedeutet,
$R^{12}$ $C_2$-$C_8$-Alkylen und
q eine Zahl von 2 bis 8 ist.

**Claims**

1. Coating material containing, as crypto light stabilizer, a compound of the formula I or II

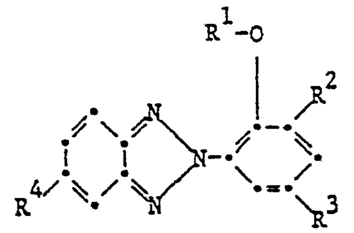

(I)

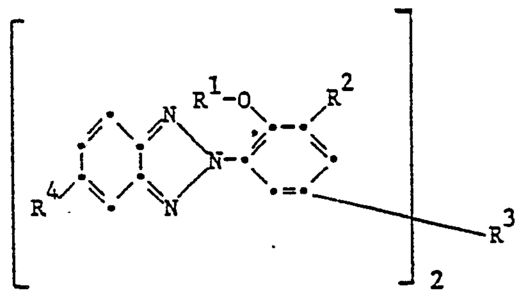

(II)

in which
$R^1$ is an acyl group of the formula $—CO—R^5$, a sulfonyl group of the formula $—SO_2—R^6$, a phosphoryl group of the formula $—P(O)_r(R^{14})(R^{15})$,
$R^2$ is $C_1$-$C_{12}$ alkyl, $C_5$-$C_{12}$ cycloalkyl, phenyl, $C_7$-$C_9$ phenylalkyl, $C_3$-$C_5$-alkenyl or halogen,
$R^3$ in formula I is $C_1$-$C_{12}$ alkyl, $C_5$-$C_{12}$ cycloalkyl, phenyl, $C_7$-$C_9$ phenylalkyl, halogen or a group of the formula $—(CH_2)_n—COOR^9$ or $—(CH_2)_nCON—(R^{10})(R^{11})$ and in formula II is a divalent radical of the formulae $—(CH_2)_n—CO—O—(CH_2)_q—O—CO—(CH_2)_n—$, $—(CH_2)_n—CO—O—(CH_2CH_2O)_p—CO—(CH_2)_n—$, $—(CH_2)_n—CO—NH—R^{12}—NH—CO—(CH_2)_n—$ or

$$-(CH_2)_n-CO-O-CH_2-\underset{OR^{13}}{\underset{|}{CH}}-CH_2-O-(CH_2)_q-O-CH_2-\underset{OR^{13}}{\underset{|}{CH}}-CH_2-O-CO-(CH_2)_n-$$

$R^4$ is hydrogen, halogen, $C_1$-$C_8$ alkyl, $C_7$-$C_9$ phenylalkyl, $C_1$-$C_8$ alkoxy or $C_2$-$C_8$ alkoxycarbonyl,
$R^5$ is $C_1$-$C_{18}$ alkyl, $C_2$-$C_{18}$ alkenyl, $—CH_2—CO—CH_3$, phenyl, phenyl substituted by $C_1$-$C_{12}$ alkyl, $C_1$-$C_4$ alkoxy or benzoyl, or is $C_7$-$C_{12}$ arylalkyl or $C_1$-$C_{12}$ alkoxy,
$R^6$ is $C_1$-$C_{12}$ alkyl, $C_6$-$C_{10}$ aryl or $C_7$-$C_{18}$ alkylaryl,
$R^9$ is hydrogen, $C_1$-$C_{12}$ alkyl or a group of the formula $—(CH_2CH_2O)_p—R^1$,
$R^{10}$ and $R^{11}$ independently of one another are hydrogen, $C_1$-$C_{12}$ alkyl, which can be interrupted by O or N, $C_5$-$C_{12}$ cycloalkyl, $C_7$-$C_9$ phenylalkyl, $C_3$-$C_5$-alkenyl, or phenyl or a 2,2,6,6-tetramethyl-4-piperidinyl radical, or $R^{10}$ and $R^{11}$ together are $C_4$-$C_6$ alkylene, $C_4$-$C_6$ oxaalkylene or $C_4$-$C_6$ azaalkylene,
$R^{12}$ is $C_1$-$C_{12}$ alkylene, which can be interrupted by 1-3 O atoms,
$R^{13}$ is $C_1$-$C_{12}$ alkyl or $C_6$-$C_{10}$ aryl,
$R^{14}$ and $R^{15}$ independently of one another are $C_1$-$C_{12}$ alkoxy, phenoxy,
$C_1$-$C_{12}$ alkyl, cyclohexyl, benzyl, phenyl or tolyl,
n is 1 or 2,

p is a number from 1 to 10,
q is a number from 2 to 12 and
r is 0 or 1.

2. Coating material according to claim 1, containing a compound of the formula I, in which
$R^1$ is a group of the formula —CO—$R^5$, —$SO_2$—$R^6$, or —P(O) ($R^{14}$) ($R^{15}$),
$R^2$ is $C_1$-$C_{12}$ alkyl, cyclohexyl or $C_7$-$C_9$ phenylalkyl,
$R^3$ is $C_1$-$C_{12}$ alkyl, cyclohexyl, $C_7$-$C_9$ phenylalkyl or a group —$CH_2CH_2COOR^9$,
$R^4$ is hydrogen , methyl or chlorine,
$R^5$ is $C_1$-$C_{12}$ alkyl, $C_2$-$C_4$ alkenyl, phenyl, benzyl or naphthylmethyl,
$R^6$ is methyl, phenyl or $C_7$-$C_{18}$ alkylphenyl and
$R^9$ is $C_1$-$C_{12}$ alkyl and
$R^{14}$ and $R^{15}$ independently of one another are $C_1$-$C_4$ alkoxy, methyl or phenyl.

3. Coating material according to claim 2, where $R^1$ is a group of the formula —CO—$R^5$ or —$SO_2$—$R^6$, and $R^5$ and $R^6$ have the meanings defined in claim 2.

4. Coating material according to claim 3, where $R^2$ is $C_1$-$C_8$ alkyl or α-dimethylbenzyl, $R^3$ is $C_1$-$C_8$ alkyl, α-dimethylbenzyl or a group —$CH_2CH_2COOR^9$, $R^4$ is hydrogen or chlorine, and $R^1$ and $R^9$ have the meanings defined in claim 3.

5. Coating material according to claim 1, containing a compound of the formula I in which $R^1$ is a group —CO—$R^5$ or —$SO_2$—$R^6$, $R^2$ is $C_1$-$C_8$ alkyl or α-dimethylbenzyl, $R^3$ is $C_1$-$C_8$ alkyl, α-dimethylbenzyl or —$CH_2CH_2COOR^9$, $R^4$ is hydrogen or chlorine, $R^5$ is $C_1$-$C_{12}$ alkyl, $C_2$-$C_4$ alkenyl, phenyl, benzyl or naphthylmethyl, $R^6$ is methyl, phenyl or $C_7$-$C_{18}$ alkylphenyl, and $R^9$ is $C_1$-$C_{12}$ alkyl.

6. Coating material according to claim 1, containing a compound of the formula II in which $R^1$ is a group of the formula —CO—$R^5$ or —$SO_2$—$R^6$, $R^2$ is $C_1$-$C_8$ alkyl or α-dimethylbenzyl, $R^3$ is a group of the formula —$CH_2CH_2COO$—$(CH_2)_q$—$OCOCH_2CH_2$— or —$CH_2CH_2CONH$—$R^{12}$—$NHCOCH_2CH_2$—, $R^4$ is hydrogen or chlorine, $R^5$ is $C_1$-$C_{12}$ alkyl, $C_2$-$C_4$ alkenyl, phenyl, benzyl or naphthylmethyl, $R^6$ is methyl, phenyl or $C_7$-$C_{18}$ alkylphenyl, $R^{12}$ is $C_2$-$C_8$ alkylene, and q is a number from 2 to 8.

7. Coating material according to claim 1, containing a binder from the series comprising alkyd resins, acrylic resins, epoxy resins, melamine resins, urea resins, polyurethanes, polyesters and phenolic resins, and mixtures thereof.

8. Coating material according to claim 1, containing as binder an oxidatively drying resin or resin mixture, and as curing catalyst an organic metal compound.

9. Use of the coating material according to claim 1 on copper or a copper alloy.

10. A compound of the formula I or II

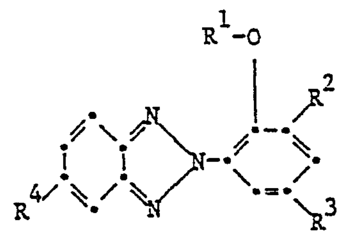

(I)

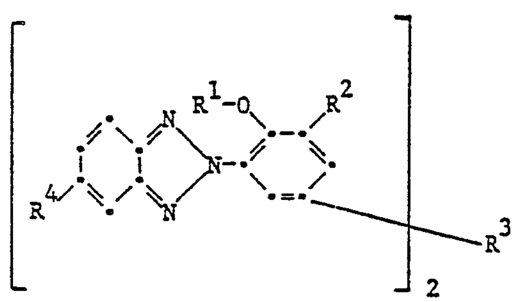

(II)

in which

$R^1$ is an acyl group of the formula —CO—$R^5$ or a sulfonyl group of the formula —$SO_2$—$R^6$ or a phosphoryl group of the formula —$P(O)_r(R^{14})$ ($R^{15}$),

$R^2$ is $C_1$-$C_{12}$ alkyl, $C_5$-$C_{12}$ cycloalkyl, phenyl, $C_7$-$C_9$ phenylalkyl, $C_3$-$C_5$-alkenyl or halogen,

$R^3$ in formula I is a group of the formula —$(CH_2)_n$—$COOR^9$ or —$(CH_2)_n$—CO—N($R^{10}$) ($R^{11}$), and in formula II is a divalent group of the formulae —$(CH_2)_n$—CO—O—$(CH_2)_q$—O—CO—$(CH_2)_n$—, —$(CH_2)_n$—CO—O—$(CH_2CH_2O)_p$—CO—$(CH_2)_n$—, —$(CH_2)_n$—CO—NH—$R^{12}$—NH—CO—$(CH_2)_n$— or

$$-(CH_2)_n-CO-O-CH_2-\underset{OR^{13}}{\underset{|}{CH}}-CH_2-O-(CH_2)_q-O-CH_2-\underset{OR^{13}}{\underset{|}{CH}}-CH_2-O-CO-(CH_2)_n-$$

$R^4$ is hydrogen, halogen, $C_1$-$C_8$ alkyl, $C_7$-$C_9$ phenylalkyl, $C_1$-$C_8$ alkoxy or $C_2$-$C_8$ alkoxycarbonyl,

$R^5$ is $C_1$-$C_{18}$ alkyl, $C_2$-$C_{18}$ alkenyl, $—CH_2—CO—CH_3$, phenyl, phenyl substituted by $C_1$-$C_{12}$ alkyl, $C_1$-$C_4$ alkoxy or benzoyl, or is $C_7$-$C_{12}$ arylalkyl or $C_1$-$C_{12}$ alkoxy,

$R^6$ is $C_1$-$C_{12}$ alkyl, $C_6$-$C_{10}$ aryl or $C_7$-$C_{18}$ alkylaryl,

$R^7$ is $C_1$-$C_{10}$ alkylene or phenylene,

$R^9$ is hydrogen, $C_1$-$C_{12}$ alkyl or a group of the formula $—(CH_2CH_2O)_p—R^1$,

$R^{10}$ and $R^{11}$ independently of one another are hydrogen, $C_1$-$C_{12}$ alkyl, which can be interrupted by O or N, $C_5$-$C_{12}$cycloalkyl, $C_7$-$C_9$ phenylalkyl, $C_3$-$C_5$ alkenyl, phenyl or a 2,2,6,6-tetramethyl-4-piperidinyl radical, or $R^{10}$ and $R^{11}$ together are $C_4$-$C_6$ alkylene, $C_4$-$C_6$ oxaalkylene or $C_4$-$C_6$ azaalkylene,

$R^{12}$ is $C_1$-$C_{12}$ alkylene which can be interrupted by 1-3 O atoms,

$R^{13}$ is $C_1$-$C_{12}$ alkyl or $C_6$-$C_{10}$ aryl,

$R^{14}$ and $R^{15}$ independently of one another are $C_1$-$C_{12}$ alkoxy, phenoxy, $C_1$-$C_{12}$ alkyl, cyclohexyl, benzyl, phenyl or tolyl,

n is 1 or 2,

p is a number from 1 to 10,

q is a number from 2 to 12, and

r is 0 or 1.

11. A compound of the formula I according to claim 10, in which

$R^1$ is a group of the formula $—CO—R^5$ or $—SO_2—R^6$,

$R^2$ is $C_1$-$C_{12}$ alkyl, cyclohexyl or $C_7$-$C_9$ phenylalkyl,

$R^3$ is a group $—CH_2CH_2COOR^9$,

$R^4$ is hydrogen, methyl or chlorine,

$R^5$ is $C_1$-$C_{12}$ alkyl, $C_2$-$C_4$ alkenyl, phenyl, benzyl or naphthylmethyl,

$R^6$ is methyl, phenyl or $C_7$-$C_{15}$ alkylphenyl.

12. A compound of the formula I according to claim 11, in which $R^1$ is a group $—CO—R^5$ or $—SO_2—R^6$, $R^2$ is $C_1$-$C_8$ alkyl or α-dimethylbenzyl, $R^4$ is hydrogen or chlorine, and $R^3$, $R^5$ and $R^6$ have the meanings defined in claim 11.

13. A compound of the formula II according to claim 10, in which

$R^1$ is a group $—CO—R^5$ or $—SO_2—R^6$,

$R^2$ is $C_1$-$C_8$ alkyl or α-dimethylbenzyl,

$R^3$ is a group of the formula $—CH_2CH_2COO—(CH_2)_q—OCOCH_2CH_2—$ or $—CH_2CH_2CONH—R^{12}—NHCOCH_2CH_2—$,

$R^4$ is hydrogen or chlorine,

$R^5$ is $C_1$-$C_{12}$ alkyl, $C_2$-$C_4$-alkenyl, phenyl, benzyl or naphthylmethyl,

$R^6$ is methyl, phenyl or $C_7$-$C_{18}$ alkylphenyl,

$R^{12}$ is $C_2$-$C_8$ alkylene, and

q is a number from 2 to 8.

## Revendications

1. Produits de revêtement qui contiennent, comme cryptostabilisant à la lumière, un composé répondant à l'une des formules I et II :

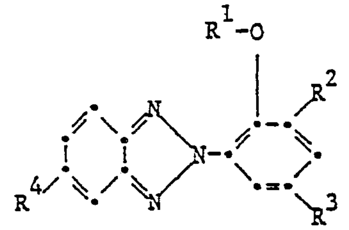

(I)

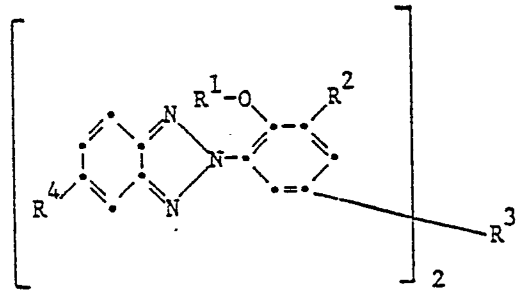

(II)

dans lesquelles :

$R^1$ représente un radical acyle de formule $-CO-R^5$, un radical sulfonyle de formule $-SO_2-R^6$ ou un radical phosphoryle de formule $-P(O)_r(R^{14})(R^{15})$,

$R^2$ représente un alkyle en $C_1$-$C_{12}$, un cycloalkyle en $C_5$-$C_{12}$, un phényle, un phénylalkyle en $C_7$-$C_9$, un alcényle en $C_3$-$C_5$ ou un halogène,

$R^3$ représente, dans la formule I, un alkyle en $C_1$-$C_{12}$, un cycloalkyle en $C_5$-$C_{12}$, un phényle, un phénylalkyle en $C_7$-$C_9$, un halogène ou un radical de formule $-(CH_2)_n-COOR^9$ ou $-(CH_2)_n-CO-N(R^{10})(R^{11})$ et, dans la formfule II, un radical bivalent répondant à l'une des formules suivantes $-(CH_2)_n-CO-O-(CH_2)_q-O-CO-(CH_2)_n-$, $-(CH_2)_n-CO-O-(CH_2CH_2O)_p-CO-(CH_2)_n-$, $-(CH_2)_n-CO-NH-R^{12}-NH-CO-(CH_2)_n-$ et

$$-(CH_2)_n-CO-O-CH_2-\underset{OR^{13}}{\underset{|}{CH}}-CH_2-O-(CH_2)_q-O-CH_2-\underset{OR^{13}}{\underset{|}{CH}}-CH_2-O-CO-(CH_2)_n-$$

$R^4$ représente l'hydrogène, un halogène, un alkyle en $C_1$-$C_8$, un phénylalkyle en $C_7$-$C_9$, un alcoxy en $C_1$-$C_8$ ou un alcoxycarbonyle en $C_2$-$C_8$,

$R^5$ représente un alkyle en $C_1$-$C_{18}$, un alcényle en $C_2$-$C_{18}$, $-CH_2-CO-CH_3$, un phényle, un phényle porteur d'un alkyle en $C_1$-$C_{12}$, d'un alcoxy en $C_1$-$C_4$ ou d'un benzoyle, un arylalkyle en $C_7$-$C_{12}$ ou un alcoxy en $C_1$-$C_{12}$,

$R^6$ représente un alkyle en $C_1$-$C_{12}$, un aryle en $C_6$-$C_{10}$ ou un alkylaryle en $C_7$-$C_{18}$,

$R^9$ représente l'hydrogène, un alkyle en $C_1$-$C_{12}$ ou un radical de formule $-(CH_2CH_2O)_p-R^1$,

$R^{10}$ et $R^{11}$ représentent chacun, indépendamment l'un de l'autre, l'hydrogène, un alkyle en $C_1$-$C_{12}$, qui peut être interrompu par O ou N, un cycloalkyle en $C_5$-$C_{12}$, un phénylalkyle en $C_7$-$C_9$, un alcényle en $C_3$-$C_5$, un phényle ou un radical tétraméthyl-2,2,6,6 pipéridyle-4 ou encore $R^{10}$ et $R^{11}$ forment ensemble un radical alkylène, oxa-alkylène ou aza-alkylène contenant de 4 à 6 atomes de carbone,

$R^{12}$ représente un alkylène en $C_1$-$C_{12}$, qui peut être interrompu par 1 à 3 atomes d'oxygène,

$R^{13}$ représente un alkyle en $C_1$-$C_{12}$ ou un aryle en $C_6$-$C_{10}$,

$R^{14}$ et $R^{15}$ représentent chacun, indépendamment l'un de l'autre, un alcoxy en $C_1$-$C_{12}$, un phénoxy, un alkyle en $C_1$-$C_{12}$, un cyclohexyle, un benzyle, un phényle ou un tolyle,

n est égal à 1 ou à 2,

p représente un nombre de 1 à 10,

q représente un nombre de 2 à 12, et

r est égal à 0 ou à 1.

2. Produit de revêtement selon la revendication 1 qui contient un composé de formule I dans lequel :

$R^1$ représente un radical de formule $-CO-R^5$, $-SO_2-R^6$ ou $-P(O)(R^{14})(R^{15})$,

$R^2$ représente un alkyle en $C_1$-$C_{12}$, un cyclohexyle ou un phénylalkyle en $C_7$-$C_9$,

$R^3$ représente un alkyle en $C_1$-$C_{12}$, un cyclohexyle, un phénylalkyle en $C_7$-$C_9$ ou un radical $-CH_2-CH_2-COOR^9$,

$R^4$ représente l'hydrogène, un méthyle ou le chlore

$R^5$ représente un alkyle en $C_1$-$C_{12}$, un alcényle en $C_2$-$C_4$, un phényle, un benzyle ou un naphtyl-méthyle,

$R^6$ représente un méthyle, un phényle ou un alkylphényle en $C_7$-$C_{18}$,

$R^9$ représente un alkyle en $C_1$-$C_{12}$ et

$R^{14}$ et $R^{15}$ représentent chacun, indépendamment l'un de l'autre, un alcoxy en $C_1$-$C_4$, un méthyle ou un phényle.

3. Produits de revêtement selon la revendication 2 dans lesquels $R^1$ représente un radical $-CO-R^5$ ou un radical $-SO_2-R^6$, dans lesquels $R^5$ et $R^6$ ont les significations qui leur ont été données à la revendication 2.

4. Produits de revêtement selon la revendication 3 dans lesquels $R^2$ représente un alkyle en $C_1$-$C_8$ ou un $\alpha$-diméthyl-benzyle, $R^3$ un alkyle en $C_1$-$C_8$, un $\alpha$-diméthylbenzyle ou un radical $-CH_2CH_2-COOR^9$, $R^4$ l'hydrogène ou le chlore, et $R^1$ et $R^9$ ont les significations qui leur ont été données à la revendication 3.

5. Produits de revêtement selon la revendication 1 qui contiennent un composé de formule I dans lequel $R^1$ représente un radical $-CO-R^5$ ou $-SO_2-R^6$, $R^2$ un alkyle en $C_1$-$C_8$ ou un $\alpha,\alpha$-diméthyl-benzyle, $R^3$ un alkyle en $C_1$-$C_8$, un $\alpha,\alpha$-diméthyl-benzyle ou un radical $-CH_2-CH_2-COOR^9$, $R^4$ représente l'hydrogène ou le chlore, $R^5$ représente un alkyle en $C_1$-$C_{12}$, un alcényle en $C_2$-$C_4$, un phényle, un benzyle ou un naphtyl-méthyle, $R^6$ représente un méthyle, un phényle ou un alkylphényle en $C_7$-$C_{18}$, et $R^9$ un alkyle en $C_1$-$C_{12}$.

6. Produits de revêtement selon la revendication 1 qui contiennent un composé de formule II dans lequel $R^1$ représente un radical $-CO-R^5$ ou $-SO_2-R^6$, $R^2$ un alkyle en $C_1$-$C_8$ ou un $\alpha,\alpha$-diméthyl-benzyle, $R^3$ un radical de formule $-CH_2CH_2COO-(CH_2)_q-OCOCH_2CH_2-$ ou $-CH_2CH_2CONH-R^{12}-NHCOCH_2CH_2-$, $R^4$ l'hydrogène ou le chlore, $R^5$ un alkyle en $C_1$-$C_{12}$, un alcényle en $C_2$-$C_4$, un phényle, un benzyle ou un naphtyl-méthyle, $R^6$ représente un méthyle, un phényle ou un alkylphényle en $C_7$-$C_{18}$, $R^{12}$ représente un alkylène en $C_2$-$C_8$ et q représente un nombre de 2 à 8.

7. Produits de revêtement selon la revendication 1 qui contiennent un liant de la série des résines alkydes, des résines acryliques, des résines époxydiques, des résines de mélamine, des résines d'urée, des polyuréthannes, des polyesters et des résines phénoliques ou de leurs mélanges.

8. Produits de revêtement selon la revendication 1 qui contiennent, comme liant, une résine ou un mélange de résines séchant par oxydation et, comme catalyseur de durcissement, un composé métallique organique.

9. Application du produit de revêtement selon la revendication 1 sur du cuivre ou sur un alliage de cuivre.

10. Composés répondant à l'une des formules I et II :

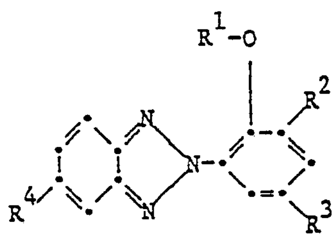

(I)

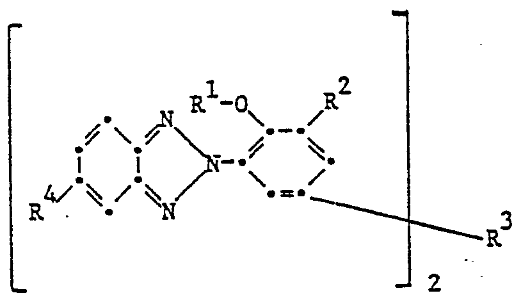

(II)

dans lesquelles :

$R^1$ représente un radical acyle de formule $—CO—R^5$, un radical sulfonyle de formule $—SO_2—R^6$ ou un radical phosphoryle de formule $—P(O)_r(R^{14})(R^{15})$,

$R^2$ représente un alkyle en $C_1$-$C_{12}$, un cycloalkyle en $C_5$-$C_{12}$, un phényle, un phénylalkyle en $C_7$-$C_9$, un alcényle en $C_3$-$C_5$ ou un halogène,

$R^3$ représente, dans la formule I, un radical de formule $—(CH_2)_n—COOR^9$ ou $—(CH_2)_n—CO—N(R^{10})(R^{11})$ et, dans la formule II, un radical bivalent répondant à l'une des formules suivantes $—(CH_2)_n—CO—O—(CH_2)_q—O—CO—(CH_2)_n—$, $—(CH_2)_n—CO—O—(CH_2CH_2O)_p—CO—(CH_2)_n—$, $—(CH_2)_n—CO—NH—R^{12}—NH—CO—(CH_2)_n—$ et

$$-(CH_2)_n-CO-O-CH_2-\underset{OR^{13}}{\underset{|}{CH}}-CH_2-O-(CH_2)_q-O-CH_2-\underset{OR^{13}}{\underset{|}{CH}}-CH_2-O-CO-(CH_2)_n-$$

$R^4$ représente l'hydrogène, un halogène, un alkyle en $C_1$-$C_8$, un phénylalkyle en $C_7$-$C_9$, un alcoxy en $C_1$-$C_8$ ou un alcoxycarbonyle en $C_2$-$C_8$,

$R^5$ représente un alkyle en $C_1$-$C_{18}$, un alcényle en $C_2$-$C_{18}$, $—CH_2—CO—CH_3$, un phényle, un phényle porteur d'un alkyle en $C_1$-$C_{12}$, d'un alcoxy en $C_1$-$C_4$ ou d'un benzoyle, un arylalkyle en $C_7$-$C_{12}$ ou un alcoxy en $C_1$-$C_{12}$,

$R^6$ représente un alkyle en $C_1$-$C_{12}$, un aryle en $C_6$-$C_{10}$ ou un alkylaryle en $C_7$-$C_{18}$,

$R^7$ représente un alkylène en $C_1$-$C_{10}$ ou un phénylène,

$R^9$ représente l'hydrogène, un alkyle en $C_1$-$C_{12}$ ou un radical de formule $—(CH_2CH_2O)_p—R^1$,

$R^{10}$ et $R^{11}$ représentent chacun, indépendamment l'un de l'autre, l'hydrogène, un alkyle en $C_1$-$C_{12}$, qui peut être interrompu par O ou N, un cycloalkyle en $C_5$-$C_{12}$, un phénylalkyle en $C_7$-$C_9$, un alcényle en $C_3$-$C_5$, un phényle ou un radical tétraméthyl-2,2,6,6 pipéridyle-4, ou encore $R^{10}$ et $R^{11}$ forment ensemble un radical alkylène, oxa-alkylène ou aza-alkylène contenant de 4 à 6 atomes de carbone,

$R^{12}$ représente un alkylène en $C_1$-$C_{12}$, qui peut être interrompu par 1 à 3 atomes d'oxygène,

$R^{13}$ représente un alkyle en $C_1$-$C_{12}$ ou un aryle en $C_6$-$C_{10}$,

$R^{14}$ et $R^{15}$ représentent chacun, indépendamment l'un de l'autre, un alcoxy en $C_1$-$C_{12}$, un phénoxy, un alkyle en $C_1$-$C_{12}$, un cyclohexyle, un benzyle, un phényle ou un tolyle et

n est égal à 1 ou à 2,

p représente un nombre de 1 à 10,

q représente un nombre de 2 à 12, et
r est égal à 0 ou à 1.

11. Composé de formule I selon la revendication 10 dans lesquels :
$R^1$ représente un radical de formule $—CO—R^5$, $—SO_2—R^6$ ou $—P(O)(R^{14})(R^{15})$,
$R^2$ représente un alkyle en $C_1$-$C_{12}$, un cyclohexyle ou un phénylalkyle en $C_7$-$C_9$,
$R^3$ représente un radical $—CH_2—CH_2—COOR^9$,
$R^4$ représente l'hydrogène, un méthyle ou le chlore,
$R^5$ représente un alkyle en $C_1$-$C_{12}$, un alcényle en $C_2$-$C_4$, un phényle, un benzyle ou un naphtylméthyle, et
$R^6$ représente un méthyle, un phényle ou un alkylphényle en $C_7$-$C_{15}$.

12. Composés de formule I selon la revendication 11 dans lesquels $R^1$ représente un radical $—CO—R^5$ ou $—SO_2—R^6$, $R^2$ représente un alkyle en $C_1$-$C_8$ ou un α,α-diméthyl-benzyle, $R^4$ représente l'hydrogène ou le chlore, et $R^3$, $R^5$ et $R^6$ ont les significations qui leur ont été données à la revendication 11.

13. Composés de formule II selon la revendication 10 dans lesquels :
$R^1$ représente un radical de formule $—CO—R^5$ ou $—SO_2—R^6$,
$R^2$ représente un alkyle en $C_1$-$C_8$, un cyclohexyle ou un α,α-diméthyl-benzyle,
$R^3$ représente un radical de formule $—CH_2CH_2COO—(CH_2)_q—OCOCH_2CH_2—$ ou de formule $—CH_2CH_2CONH—R^{12}—NHCOCH_2CH_2—$,
$R^4$ représente l'hydrogène ou le chlore,
$R^5$ représente un alkyle en $C_1$-$C_{12}$, un alcényle en $C_2$-$C_4$, un phényle, un benzyle ou un naphtylméthyle,
$R^6$ représente un méthyle, un phényle ou un alkylphényle en $C_7$-$C_{18}$,
$R^{12}$ représente un alkylène en $C_2$-$C_8$ et
q représente un nombre de 2 à 8.